# FASCICULE DE BREVET EUROPEEN

(11) **EP 4 281 126 B1**
(45) Date de publication et mention de la délivrance du brevet: **26.03.2025**
(21) Numéro de dépôt: 22706878.0
(22) Date de dépôt: 02.02.2022
(51) Int. Cl.: A61L 9/20, A41D 13/11, A61M 16/06, A62B 18/02

(54) **PROCÉDÉ ET DISPOSITIF DE DÉSINFECTION D'AIR RESPIRÉ PAR UNE PERSONNE**
VERFAHREN UND VORRICHTUNG ZUR DESINFEKTION DER VON EINER PERSON ANGESAUGTEN LUFT
METHOD AND DEVICE FOR DISINFECTING THE AIR BREATHED IN BY A PERSON

(30) Priorité: 15.02.2021 FR 2101409
(43) Date de publication de la demande: 29.11.2023
(73) Titulaire: Brune, Jean-Pierre, 83160 La Valette (FR)
(72) Inventeur: SALESCO BRUNE, Magali, 83210 Sollies-Pont (FR); BRUNE, Caroline, 83210 Sollies-Pont (FR); BRUNE, Jean-Pierre, 83160 La Valette (FR)
(74) Mandataire: Marchand, André
(86) Numéro de dépôt international: PCT/FR2022/050201
(87) Numéro de publication internationale: WO 2022/171949

(56) Documents cités:
- WO-A1-03/092748
- CN-A- 111 053 979
- CN-A- 111 194 955

## Description

La présente invention concerne un dispositif de de désinfection de l'air associé à un masque respiratoire bucco-nasal de protection contre les infections, et un procédé pour désinfecter l'air respiré par une personne.

Les ultraviolets (UV) et notamment ceux situés dans la bande de longueurs d'onde des UV-C (240 à 280 nm) agissent contre les virus et bactéries en les tuant ou en les rendant inaptes à infecter une personne. C'est pourquoi on trouve des lampes à UV dans de nombreux dispositifs de purification de l'air et de l'eau, ou dans des enceintes de désinfection d'objets, tels que des lunettes ou des téléphones.

On a également proposé d'intégrer une ou plusieurs lampes UV dans un masque respiratoire bucco-nasal de protection contre les infections, pour éradiquer les virus et bactéries. Il s'avère que l'intensité lumineuse du rayonnement UV émis et le temps d'exposition aux UV sont insuffisants pour avoir un réel effet sur l'air respiré à travers un masque respiratoire. En réalité, les ultraviolets émis n'ont qu'un effet sur les germes présents à la surface du masque.

Des exemples sont divulgués dans CN111194955 A, WO03092748 A1 et CN111053979 A.

Les doses d'ultraviolets nécessaires pour éradiquer les virus et bactéries dans un volume d'air dépendent de plusieurs facteurs, à savoir la longueur d'onde et l'intensité lumineuse du rayonnement UV reçu, ainsi que le temps d'exposition, et plus exactement, la dose reçue qui est égale au produit de l'intensité lumineuse du rayonnement UV par le temps d'exposition. En outre, il est généralement admis que la survivance des germes à l'exposition aux ultraviolets suit un modèle de décroissance exponentielle, de la forme S = e^{-kD}, où S représente la fraction de germes encore présents après l'exposition aux ultraviolets, D représente la dose appliquée, et k un coefficient qui est propre à chaque germe.

Par ailleurs, les débits respirés varient dans des proportions importantes selon que la personne est au repos ou exerce une activité physique, et d'une personne à l'autre.

Il est donc souhaitable de proposer un dispositif qui soit efficace pour désinfecter un volume très variable d'air inspiré ou expiré par une personne. Il est également souhaitable que ce dispositif puisse être porté facilement par une personne, sans entraver ses mouvements quotidiens.

Des modes de réalisation concernent un procédé de désinfection de l'air respiré par un utilisateur, le procédé comprenant des étapes consistant à : soumettre l'intérieur d'une première chambre en communication avec un volume d'air extérieur, à une première source d'un rayonnement ultraviolet ayant une longueur d'onde apte à détruire des germes, soumettre l'intérieur d'une seconde chambre en communication avec le volume d'air extérieur, à une seconde source du rayonnement ultraviolet, détecter des instants de début de phases respiratoires d'inspiration et d'expiration d'air effectuées par l'utilisateur, chaque phase respiratoire étant associée à un sens direct ou inverse de circulation d'air dans une conduite durant la phase respiratoire, durant une première phase respiratoire de sens direct suivant une première phase respiratoire de sens inverse, mettre en circulation l'air de la première chambre entre un volume intérieur d'un masque respiratoire couvrant de manière étanche la bouche et le nez de l'utilisateur et le volume d'air extérieur, la première phase respiratoire de sens direct étant suivie d'une seconde phase respiratoire de sens inverse, et durant une seconde phase respiratoire de sens direct suivant la seconde phase respiratoire de sens inverse, mettre en circulation l'air de la seconde chambre entre le volume intérieur du masque respiratoire et le volume d'air extérieur.

Selon un mode de réalisation, le procédé comprend une étape de détection que l'air de la première chambre est totalement renouvelé avant la fin de la première phase respiratoire de sens direct, suivie d'une étape de mise en circulation de l'air de la seconde chambre en alternance avec la mise en circulation de l'air de la première chambre, entre le volume intérieur du masque respiratoire et le volume d'air extérieur.

Selon un mode de réalisation, le procédé comprend plusieurs étapes en alternance de mise en circulation de l'air de la première chambre et de mise en circulation de l'air de la seconde chambre, entre le volume intérieur du masque respiratoire et le volume d'air extérieur, durant la première phase respiratoire de sens direct, chaque alternance étant déclenchée par la détection que l'air est totalement renouvelé dans la première ou seconde chambre.

Selon un mode de réalisation, le procédé comprend des étapes d'extinction des première et seconde sources de rayonnement ultraviolet pendant chaque phase respiratoire de sens inverse.

Selon un mode de réalisation, les première et seconde phases respiratoires de sens direct sont des phases d'inspiration de l'utilisateur, et les première et secondes phase respiratoires de sens inverse sont des phases d'expiration de l'utilisateur, ou bien les première et seconde phases respiratoires de sens direct sont des phases d'expiration de l'utilisateur, et les première et secondes phase respiratoires de sens inverse sont des phases d'inspiration de l'utilisateur.

Selon un mode de réalisation, le procédé comprend des étapes d'activation d'un ventilateur pour faire circuler l'air durant chaque phase respiratoire de sens direct.

Des modes de réalisation peuvent également concerner un dispositif de désinfection d'air respiré par un utilisateur, le dispositif comprenant : une première chambre et une seconde chambre, en communication avec un volume d'air extérieur et couplées par un dispositif de vanne commandé, à une première conduite reliée au volume intérieur d'un masque respiratoire, une source de rayonnement ultraviolet, installée à l'intérieur chacune des première et seconde chambres, et un dispositif de mesure pour déterminer un sens de circulation de l'air dans la première conduite, le dispositif de désinfection étant configuré pour mettre en œuvre le procédé défini précédemment, la détection des instants de début de phases respiratoires d'inspiration et d'expiration d'air étant effectuées à l'aide du dispositif de mesure, et les mises en circulation de l'air dans la première chambre et dans la seconde chambre étant effectuées à l'aide du dispositif de vanne.

Selon un mode de réalisation, chacune des première et seconde chambres comprend un dispositif de ventilation pour faire circuler l'air au travers de la chambre.

Selon un mode de réalisation, le dispositif de ventilation comprend une pompe ou un ventilateur et/ou met en œuvre l'effet Coanda.

Selon un mode de réalisation, la source de rayonnement UV dans chacune des première et seconde chambres comprend une lampe à tube à quartz UV ou des LED UV.

Selon un mode de réalisation, le dispositif est installé dans un logement portatif, incluant une source d'alimentation électrique autonome.

Selon un mode de réalisation, chacune des première et seconde chambre est associée à un compresseur pour augmenter la pression dans la chambre.

Selon un mode de réalisation, la source de rayonnement UV est installée dans chacune des première et seconde chambres et configurée pour soumettre un volume d'exposition dans la chambre à une intensité lumineuse supérieure ou égale à 10 mW/cm².

Des exemples de réalisation de l'invention seront décrits dans ce qui suit, à titre non limitatif en relation avec les figures jointes parmi lesquelles :
[Fig. 1] la figure 1 représente schématiquement un dispositif de désinfection, selon un mode de réalisation,
[Fig. 2] la figure 2 représente schématiquement un élément du dispositif de désinfection, selon un mode de réalisation,
[Fig. 3] [Fig. 4] [Fig. 5] les figures 3 à 5 sont des chronogrammes schématiques de variation du débit d'air respiré, illustrant le fonctionnement du dispositif de désinfection, selon un divers modes de réalisation.

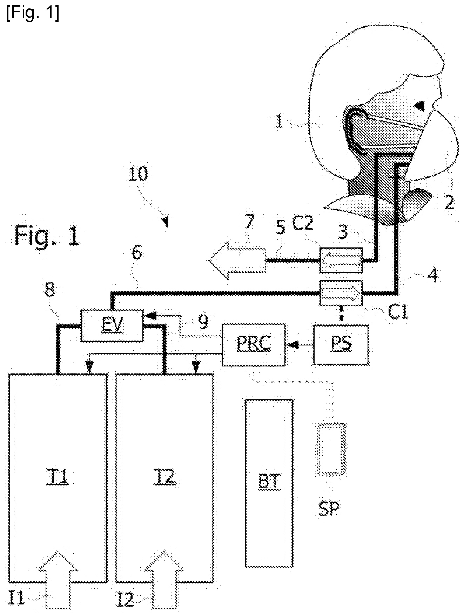

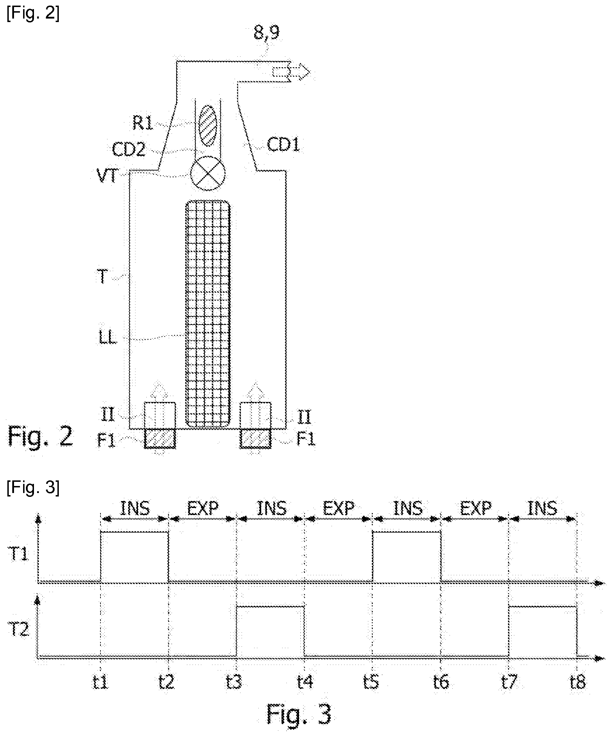

La figure 1 représente un dispositif 10 de désinfection d'air respiré, selon un mode de réalisation. Le dispositif 10 comprend deux chambres T1, T2, un dispositif de vanne EV commandé électriquement, un capteur de pression PS, deux clapets anti-retour C1, C2, et un processeur PRC recevant des signaux de mesure de pression du capteur de pression PS et commandant le dispositif de vanne EV et les chambres T1, T2. Le dispositif 10 comprend également un masque respiratoire bucco-nasal 2 étanche, agencé pour être fixé sur la tête de l'utilisateur 1 de manière à couvrir de manière étanche la bouche et le nez de l'utilisateur. Le volume intérieur du masque 2 est relié aux clapets C1, C2 par deux conduites 3, 4. Le clapet C2 est relié à une entrée 7 du dispositif par une conduite 5, l'entrée 7 étant ouverte à l'air ambiant. Le clapet C2 peut être intégré directement dans le masque 2. Le clapet C1 et relié au le dispositif de vanne EV par une conduite 6, le dispositif EV étant relié aux chambres T1, T2 par des conduites 8, 9, respectives. Le capteur de pression PS mesure la pression dans la conduite 4 par exemple dans le clapet C1. Le dispositif de vanne EV est agencé de manière à relier la conduite 6 soit à la conduite 8 vers la chambre T1, soit à la conduite 9 vers la chambre T2. Chaque chambre T1, T2 comprend une entrée I1, I2 ouverte à l'air ambiant, et une source de rayonnement UV (figure 2). Chaque chambre T1, T2 peut également comprendre un ventilateur pour faciliter la circulation d'air dans le dispositif et éviter d'imposer un effort supplémentaire à l'utilisateur 1 pour respirer. Le dispositif 10 comprend également une ou plusieurs batteries BT pour alimenter le processeur PRC, le capteur PS, le dispositif EV et les chambres T1, T2 (notamment la source de rayonnement).

L'ensemble des chambres T1, T2, de la batterie BT, du dispositif de vanne EV et du processeur PRC, peut être disposé dans un logement (coffret ou sac) associé à des moyens de fixation pour suspendre le logement au cou ou aux épaules de l'utilisateur 1, ou encore pour le maintenir autour de la taille de l'utilisateur. La batterie BT peut être disposée dans un boitier séparé, par exemple fixé à la taille de l'utilisateur.

Lorsque l'utilisateur 1 expire de l'air, le clapet C2 s'ouvre, tandis que le clapet C1 reste fermé. L'air expiré est donc expulsé par l'ouverture 7. Lorsque l'utilisateur inspire de l'air, le clapet C1 s'ouvre, tandis que le clapet C2 reste fermé. Le processeur PRC commande le dispositif EV de manière à mettre en communication la conduite 6 avec la chambre T1 et la chambre T2, alternativement. Ainsi, l'air inspiré provient soit de la chambre T1, soit de la chambre T2, laquelle se remplit au fur et à mesure, par son entrée I1, I2.

Le sens de circulation de l'air dans les conduites 6, 8 et 9, et dans les chambres T1, T2 peut être inversé, par exemple pour désinfecter l'air expiré par l'utilisateur 1, en inversant les raccordements des conduites 5, 6 aux clapets C1, C2.

Le capteur de pression PS peut être un capteur piézo-résistif intégré dans un composant à semiconducteur comportant une entrée et une sortie de gaz, et fournissant une tension analogique proportionnelle au débit de gaz entre l'entrée et la sortie. Le capteur PS peut être monté en dérivation avec le clapet C1. Le capteur de pression PS peut également comporter une roue à aubes ou une hélice disposée dans la conduite 4 ou 6 et associée à un dispositif de mesure de la vitesse de la roue ou hélice. La mesure de cette vitesse permet de déterminer la vitesse de l'écoulement de l'air dans la conduite et donc le débit d'air.

Le dispositif de vanne EV peut être de type électrovanne à trois voies et deux positions. Ainsi, l'électrovanne peut comprendre un électroaimant qui, au repos, maintient la liaison ouverte vers la chambre T1 (liaison entre les conduites 6 et 8 ouverte et liaison entre les conduites 6 et 9 fermée), et sous tension, maintient la liaison ouverte sur la chambre T2 (liaison entre les conduites 6 et 9 ouverte et liaison entre les conduites 6 et 8 fermée). Le dispositif de vanne EV peut comprendre deux électrovannes à deux voies disposées respectivement entres les conduites 6 et 8 et entre les conduites 6 et 9. Ces deux électrovannes peuvent être montées de manière à ouvrir la liaison entre les deux conduites 6, 8 ou 6, 9 lorsqu'elles sont alimentées, et fermer la liaison dans le cas contraire.

Les clapets anti-retour C1, C2 peuvent comporter une valve mobile ou être de type valve de Tesla permettant à un fluide de s'écouler dans seul sens sans pièce mobile.

Le processeur PRC peut être de type microprocesseur ou microcontrôleur.

Le masque 2 comprend une coque étanche à l'air couvrant de manière étanche le nez la bouche et le menton de l'utilisateur. La coque peut être rigide, transparente et de faible épaisseur, par exemple inférieure ou égale à 2 mm. La coque peut être fabriquée à partir d'une feuille de rhodoïd. L'étanchéité entre la coque et le visage de l'utilisateur 1 peut être obtenue par un joint en élastomère souple. Pour assurer une bonne étanchéité, la forme de la coque peut être adaptée à la forme du visage de l'utilisateur. La coque peut être maintenue sur le visage de l'utilisateur par des bandes élastiques passant derrière les oreilles ou derrière la tête, ou bien par des branches de lunettes.

Les conduites 3, 4 peuvent déboucher dans la coque ou être couplées à une conduite unique souple débouchant dans la coque, et par exemple fixée à l'une des branches de lunettes. Les conduites 3, 4 ou la conduite unique peut déboucher dans le masque par une ouverture réalisée au niveau des joues par une double peau laissant un passage de plusieurs millimètres de large.

L'adaptation de la forme de la coque à la forme du visage de l'utilisateur peut être réalisée à l'aide d'un modèle 3D du visage. Le modèle 3D peut être utilisé pour sélectionner un masque dans un ensemble de masques préfabriqués, une adaptation finale du masque préfabriqué pouvant etre effectuée par exemple par thermoformage. Alternativement, le masque peut être fabriqué à partir d'un moule réalisé par exemple à l'aide de bandes plâtrées posées sur le visage.

Le masque peut être équipé d'un microphone relié à un amplificateur couplé à un haut-parleur, pour compenser le fait que le masque peut étouffer la voix. Lorsque l'utilisateur parle, son débit respiratoire peut être perturbé. Pour en tenir compte, le processeur PRC peut être connecté au microphone.

La figure 2 représente une chambre T, selon un mode de réalisation, les chambres T1, T2 pouvant être identiques à la chambre T. La chambre T présente une forme allongée et comportant, à une extrémité, une ouverture II ouverte sur l'air ambiant, qui peut être équipée d'un filtre F1, à une extrémité opposée une zone de transition CD1 entre la chambre T et la conduite 8 ou 9, et entre ces deux extrémités, une zone d'exposition de l'air présent dans la chambre aux ultraviolets, la zone d'exposition comportant une source LL de rayonnement UV. La forme de la chambre T est adaptée à la forme de la source LL de manière à assurer un éclairement sensiblement uniforme du volume de la zone d'exposition. Les parois internes de la chambre peuvent être recouvertes d'un revêtement réfléchissant. La zone de transition CD2 peut comporter un ventilateur VT facilitant l'inspiration par l'utilisateur 1, la vitesse du ventilateur étant commandée par le processeur PRC en fonction de la pression mesurée par le capteur de pression PS.

Selon un mode de réalisation, le ventilateur VT est disposé dans ou à l'extrémité d'une canalisation CD2 fixée coaxialement dans la zone de transition CD1, l'intérieur de la canalisation CD1 étant partiellement obturé par une pièce R1 en forme d'ellipsoïde fixée coaxialement dans la canalisation CD1. L'air qui sort de la zone d'exposition de la chambre T est aspiré par le ventilateur VT dans le conduit CD2 et l'air qui transite dans la zone de transition CD1 autour de la conduite CD2 produit un effet multiplicateur par effet Coanda, ce qui permet d'obtenir un flux d'air important en sortie de la chambre dans la canalisation 8, 9, même avec un ventilateur peu puissant.

Dans l'exemple de la figure 2, la source LL présente une forme cylindrique. Ainsi, la source LL peut par exemple être de type tube à quartz basse pression produisant un rayonnement centré sur 254 nm (exactement 253,7 nm), ces longueurs d'onde ayant une action germinicide. En outre, le quartz du tube présente l'avantage de filtrer la longueur d'onde de 185 nm qui réagit avec l'oxygène de l'air pour produire de l'ozone qui est toxique. La zone d'exposition de la chambre T peut également présenter une forme cylindrique, la source LL étant fixée coaxialement à la zone d'exposition dans la chambre T. L'ouverture II, ainsi que le filtre F1 peuvent alors présenter une forme annulaire autour de la source LL, comme représenté sur la figure 2.

La chambre T peut présenter toute autre forme, notamment une forme adaptée à son transport, par exemple une forme parallélépipédique. En outre, la chambre T peut présenter plusieurs ouvertures. Par ailleurs, des chicanes peuvent être prévues autour de l'ouverture II afin d'éviter que le rayonnement UV puisse sortir de la chambre T, sachant que les UV-C sont dangereux pour les yeux et la peau.

La source LL peut également être à base de diodes électroluminescentes (LED), le nombre et la disposition des LED dans la zone d'exposition et la forme de cette dernière étant adaptés en conséquence pour obtenir un éclairement sensiblement uniforme du volume de la zone d'exposition. Les LED à UV peuvent produire un rayonnement centré sur 265 nm. Les LED UV ayant tendance à dégager beaucoup de chaleur, elles peuvent être couplées à un caloduc ou à un module à effet Peltier, ou encore être associées à un ventilateur.

Le ventilateur VT peut être remplacé par ou être associé à une pompe unique pour les deux chambres T1, T2, par exemple placée dans la conduite 6. L'entrée II de chacune des deux chambres T1, T2 peut être équipée d'un clapet anti-retour.

Les figures 3 à 5 sont des chronogrammes de variation du débit d'air respiré dans le dispositif 10 de désinfection d'air. La figure 3 illustre plus particulièrement le cas où le volume d'air présent dans chacune des chambres est suffisant pour alimenter une inspiration. La figure 3 montre des phases d'inspiration INS délimitées par les instants t1-t2, t3-t4, t5-t6 et t7-t8 en alternance avec des phases d'expiration EXP de l'utilisateur, délimitées par les instants t2-t3, t4-t5, t6-t7.

Avant l'instant t1, le vanne EV clapet C1 est fermé. Les deux chambres T1, T2 ne sont donc pas en communication avec le masque 2, l'air dans les chambres étant soumis au rayonnement UV.. A l'instant t1, l'utilisateur commence à inspirer de l'air, ce qui déclenche l'ouverture du clapet C1. Le processeur PRC détecte alors une dépression (mesure de pression inférieure à une première valeur de seuil) et donc commande le dispositif de vanne EV pour mettre en communication les conduites 6 et 8 (chambre T1), et déclenche le ventilateur VT pour aspirer l'air dans la chambre T1 à une vitesse ajustée en fonction des mesures de pression fournies par le capteur PS.

Entre les instants t2 et t3 (ainsi qu'à chaque phase d'expiration EXP), l'utilisateur expire de l'air, ce qui ferme le clapet C1 et ouvre le clapet C2. La pression dans la conduite 4 passe au-dessus d'une seconde valeur de seuil. Le processeur PRC commande alors l'arrêt du ventilateur VT dans la chambre T1.

A l'instant t3, le processeur PRC détecte à nouveau que l'utilisateur inspire de l'air grâce au capteur PS, et commande le dispositif de vanne EV pour mettre en communication les conduites 6 et 9 (chambre T2). Le processeur PRC met également en marche le ventilateur VT en fonction de la pression mesurée par le capteur PS. Ainsi, les phases d'inspiration INS sont effectuées alternativement au travers des chambres T1, T2. Il en résulte que durant la période entre les instants t2 et t5 s'étendant sur deux phases d'expiration EXP et une phase d'inspiration INS, l'air dans la chambre T1 n'est pas renouvelé et donc reste exposé aux UV. De même, durant la période entre les instants t4 et t7, s'étendant sur deux phases d'expiration EXP et une phase d'inspiration INS, l'air dans la chambre T2 n'est pas renouvelé et reste exposé aux UV.

Selon un exemple de réalisation, la source LL de rayonnement UV est un tube à quartz basse pression présentant une longueur de 12,5 cm et un diamètre de 3 cm, et émettant une puissance lumineuse de 3 W. L'intensité lumineuse à la surface du tube peut être estimée à 8,4 mW/cm². Si la chambre T1, T2 présente un volume de 0,5 l calculé en excluant le volume du tube, et le tube étant positionné au centre de la chambre, l'intensité lumineuse du rayonnement UV appliqué au volume d'air dans la chambre T1, T2 est de l'ordre de 10 mW/cm².

L'efficacité des ultraviolets contre un germe est généralement mesurée par la dose D₉₀ nécessaire pour éradiquer 90% du germe. Cette valeur dépend de la nature du germe et du milieu dans lequel le germe est présent. Différentes études montrent que les virus et bactéries sont très sensibles aux UV, surtout les virus dans l'air sec et un peu moins dans l'air humide. Selon différents auteurs, les coronavirus nécessitent des doses allant de 7 à 2410 J/m² pour atteindre un abattement de 90 %. Des études sur le virus SRAS-CoV-2 (Bianco et Al., "UV-C irradiation is highly effective in inactivating SARS-CoV-2 replication", 2020, doi: https://doi.org/10.1101/2020.06.05.20123463) établissent que la valeur moyenne de la dose D₉₀ est de 2,7 mJ/cm², et suggèrent qu'une dose égale à 4,7 mJ/cm² serait efficace contre tous les coronavirus.

Une personne au repos inspire et expire en moyenne 0,5 l et effectue 12 à 16 cycles respiratoires par minute. Il en résulte un débit respiratoire de 8 l/min et une durée d'un cycle respiratoire minimum de 3,8 s. Une inspiration ou expiration (entre les instants ti et ti+1, avec i = 1, 2, 3, ...) dure donc 1,9 s. Les chambres T1 et T2 peuvent donc présenter un volume au moins égal à 0,5 l. L'air dans chaque chambre T1, T2 reste donc exposé aux UV pendant 3 fois 1,9 s, soit 5,7 s, en omettant les intervalles de temps éventuels entre les phases respiratoires d'inspiration et d'expiration.

Une intensité lumineuse de 10 mW/cm² permet d'atteindre une dose de 57 mJ/cm² pendant une phase d'exposition (entre les instants t2 et t5 ou entre les instants t4 et t7) de 5,7 s, soit une valeur bien supérieure à la dose de 4,7 mJ/cm² efficace pour éliminer 90% des coronavirus. La loi de variation du nombre de germes en fonction du temps étant logarithmique, un doublement du temps d'exposition permet de détruire dix fois plus de germes.

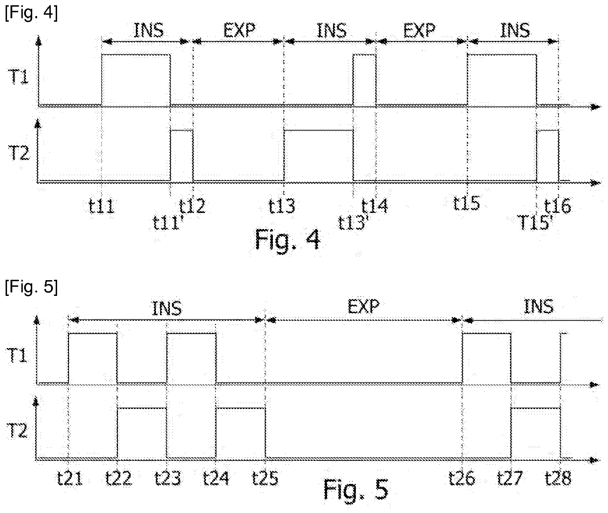

La figure 4 illustre le fonctionnement du dispositif 10 de désinfection d'air lorsque l'activité physique de l'utilisateur 1 est plus soutenue, c'est-à-dire, lorsque le volume d'air inspiré à chaque inspiration INS dépasse le volume d'air dans chacune des chambres T1, T2. Dans l'exemple de la figure 4, le volume de chaque chambre T1, T2 est de 0,5 l, le débit respiratoire de l'utilisateur est de 12 l/min, atteint en 17 cycles de respiration par minute. Le volume d'air par inspiration est donc de 0,7 l et la durée d'inspiration INS ou d'expiration EXP est de 1,76 s. Chaque phase d'inspiration INS et d'expiration EXP s'étend entre des instants ti et ti+1, i = 11, 12, 13, 14, 15).

Avant l'instant t11, l'air présent dans les chambres T1, T2 est désinfecté, et le clapet C1 est dans sa position fermée. A l'instant t11, le dispositif de vanne EV est commandé pour mettre la chambre T1 en communication avec la conduite 6. Entre les instants t11 et t11', l'air présent dans la chambre T1 est totalement aspiré. A l'instant t11', le processeur PRC détermine que tout l'air désinfecté dans la chambre T1 a été aspiré en fonction de la mesure de pression fournie par le capteur PS (qui permet de calculer le débit et donc le volume aspiré), et commande le dispositif de vanne EV pour mettre en communication la chambre T2 avec la conduite 6. Entre les instants t11' et t12, la phase d'inspiration se poursuit, une partie de l'air désinfecté présent dans la chambre T2 étant inspirée par l'utilisateur. Une partie de l'air présent dans la chambre T2 a donc été renouvelée entre les instants t11' et t12. L'utilisateur passe en phase d'expiration EXP entre les instants t12 et t13. A la phase d'inspiration INS suivante, entre les instants t13 et t14, le processeur PRC commande le dispositif de vanne EV pour fournir à l'utilisateur 1 la totalité de l'air désinfecté présent dans la chambre T2, puis une partie de l'air désinfecté présent dans la chambre T1, entre les instants t13' et t14. Ensuite, le dispositif 10 passe en phase d'expiration EXP entre les instants t14 et t15. Entre les instant t15 et t16, le dispositif passe à nouveau en phase d'inspiration INS qui se déroule de la même manière qu'entre les instants t11 et t12.

La durée d'exposition aux ultraviolets de l'air dans chacune des chambres T1, T2 est supérieure à trois phases d'inspiration ou expiration (entre les instants t11' et t15 pour la chambre T1), soit 5,3 s. Cependant, pendant ces trois phases, une partie de l'air (0,2 l) a été renouvelée dans la chambre T1, entre les instants t13' et t14, et a été exposée aux UV durant une seule phase d'expiration EXP, entre les instants t14 et t15. Cette partie d'air renouvelé a donc reçu une dose de 17,6 mJ/cm² (10 x 1,76).

Lorsque le volume restant à aspirer est faible à l'instant t11', compte tenu de la phase respiratoire précédente, il peut être prévu de laisser la chambre T1 en communication avec la conduite 6, sachant que l'air qui entre par l'orifice II à la base de la chambre T1, traverse toute la longueur de celle-ci et est donc soumis aux UV.

La figure 5 illustre le fonctionnement du dispositif 10 de désinfection d'air lorsque l'activité physique de l'utilisateur 1 est encore plus soutenue. Dans l'exemple de la figure 5, l'utilisateur 1 effectue 30 cycles respiratoires par minute et inspire 60 l/min (soit sept fois le volume au repos). Chaque phase d'inspiration et d'expiration, entre les instants t21 et t25, et les instants t25 et t26, dure donc 1 s, et le volume d'air inspiré ou expiré à chaque inspiration ou expiration est de 2 l.

Entre les instants t21 et t22, l'ensemble de l'air de la chambre T1 est aspiré. A l'instant t22, le processeur PRC détermine que tout l'air désinfecté de la chambre T1 a été aspiré en fonction de la mesure de pression fournie par le capteur PS, et commande le dispositif de vanne EV pour mettre en communication la chambre T2 avec la conduite 6. A l'instant t23, le processeur PRC détermine que tout l'air désinfecté de la chambre T2 a été aspiré, et commande le dispositif de vanne EV pour mettre en communication la chambre T1 avec la conduite 6. Ce processus est répété une fois entre les instants t23 et t25 pour atteindre la fin de la phase d'inspiration INS. Ainsi, entre les instants T21 et t25, le dispositif 10 procède à quatre phases d'inspiration effectuées alternativement dans chaque chambre T1, T2, pour fournir les 2 l inspirés par l'utilisateur, pendant la phase d'inspiration INS de durée 1 s. Chaque phase d'inspiration entre les instants ti et ti+1 (i=21, 22, ... 24), réalisée pendant la phase d'inspiration INS dure donc 0,25 s. La phase d'expiration EXP s'étend sur une seconde entre les instants t25 et t26, la phase d'exposition de la chambre T1 se déroulant entre les instants t24 et t26, et la phase d'exposition de la chambre T2 se déroulant entre les instants t25 et t27. Le processus exécuté durant les instants t21 à t25 est exécuté à nouveau à partir de l'instant t26.

Dans l'exemple précédent, dans lequel la source lumineuse LL produit une intensité lumineuse de 10 mW/cm², une durée d'exposition de 0,5 s permet d'atteindre une dose de 5 mJ/cm² qui est encore supérieure à la dose de 4,7 mJ/cm² efficace pour éliminer notamment 90% des coronavirus.

Dans le cas où le sens de circulation de l'air dans les conduites 6, 8 et 9, et dans les chambres T1, T2 est inversé, les phases d'inspiration INS et d'expiration EXP dans les figures 3, 4 et 5 sont inversées.

Selon un mode de réalisation, l'air introduit dans les chambres T1, T2 est comprimé au moyen d'un compresseur d'air, pour augmenter la capacité de désinfection du dispositif, sans augmenter le volume de ce dernier, ou réduire ce volume en conservant une même capacité de désinfection d'air. Le compresseur d'air peut être couplé aux entrées I1, I2 des chambres T1, T2 dans le cas où le dispositif est utilisé pour désinfecter l'air inspiré. Dans ce cas, le dispositif de vanne EV est configuré pour pouvoir fermer en même temps les conduites 8 et 9 vers les chambres T1, T2. Le compresseur d'air peut être couplé aux conduites 8, 9 reliées aux chambres T1, T2 dans le cas où le dispositif est utilisé pour désinfecter l'air expiré, les entrées I1, I2 étant équipées de clapets anti-retour ou de vannes commandées pour maintenir les chambres T1, T2 fermées lorsque le compresseur élève la pression dans les chambres.

Le processeur PRC peut être couplé à un circuit de communication, par exemple de type Bluetooth, pour mettre en communication le processeur à un terminal SP externe tel qu'un téléphone mobile. Le processeur PRC peut alors être configuré pour transmettre au terminal SP des données, telles que des mesures relatives à la respiration de l'utilisateur (volumes, débit, fréquence, ...), des pourcentages théoriques de germes détruits, un état de charge de la batterie BT, un nombre d'heures d'utilisation du dispositif, etc.

Il apparaîtra clairement à l'homme de l'art que la présente invention est susceptible de diverses variantes de réalisation et diverses applications. En particulier, l'invention n'est pas limitée à un dispositif dans lequel les sources de rayonnement UV sont actives en permanence. En effet, il peut être souhaitable d'éteindre les sources de rayonnement UV pendant les phases où l'air n'est pas renouvelé dans les chambres T1, T2, c'est-à-dire pendant les phases d'inspiration dans l'exemple de la figure 2, ou pendant les phases d'expiration lorsque le sens de circulation de l'air dans les chambres est inversé pour désinfecter l'air expiré par l'utilisateur. En effet, certaines sources comme les LED ont tendance à s'échauffer. Il peut également être souhaitable de prolonger la durée d'utilisation du dispositif sans avoir à changer ou à recharger la batterie BT. En outre, il peut être suffisant d'exposer l'air dans les chambres au rayonnement UV uniquement pendant les phases d'inspiration ou d'expiration. La puissance lumineuse émise par la source LL pendant la durée de chacune des phases d'inspiration ou d'expiration peut être suffisante pour détruire les germes visés en fonction du débit d'air.

Le dispositif de désinfection d'air peut comporter davantage de deux chambres en parallèle, notamment pour pouvoir traiter un débit d'air plus important.

## Revendications

1. Procédé de désinfection de l'air respiré par un utilisateur, le procédé comprenant des étapes consistant à :
soumettre l'intérieur d'une première chambre (T1) en communication (11) avec un volume d'air extérieur, à une première source (LL) d'un rayonnement ultraviolet ayant une longueur d'onde apte à détruire des germes,
soumettre l'intérieur d'une seconde chambre (T2) en communication (11) avec le volume d'air extérieur, à une seconde source (LL) du rayonnement ultraviolet,
détecter des instants (t1-t8) de début de phases respiratoires d'inspiration (INS) et d'expiration (EXP) d'air effectuées par l'utilisateur, chaque phase respiratoire étant associée à un sens direct ou inverse de circulation d'air dans une conduite (4, 6) durant la phase respiratoire,
durant une première phase respiratoire de sens direct suivant une première phase respiratoire de sens inverse, mettre en circulation l'air de la première chambre (T1) entre un volume intérieur d'un masque respiratoire (2) couvrant de manière étanche la bouche et le nez de l'utilisateur (1) et le volume d'air extérieur, la première phase respiratoire de sens direct étant suivie d'une seconde phase respiratoire de sens inverse, et
**caractérisé par**,
durant une seconde phase respiratoire de sens direct suivant la seconde phase respiratoire de sens inverse, mettre en circulation l'air de la seconde chambre (T2) entre le volume intérieur du masque respiratoire (2) et le volume d'air extérieur.

2. Procédé selon la revendication 1, comprenant une étape de détection que l'air de la première chambre (T1) est totalement renouvelé avant la fin (t12, t25) de la première phase respiratoire de sens direct , suivie d'une étape de mise en circulation de l'air de la seconde chambre (T2) en alternance avec la mise en circulation de l'air de la première chambre (T1), entre le volume intérieur du masque respiratoire (2) et le volume d'air extérieur.

3. Procédé selon la revendication 1 ou 2, comprenant plusieurs étapes en alternance de mise en circulation de l'air de la première chambre (T1) et de mise en circulation de l'air de la seconde chambre (T2), entre le volume intérieur du masque respiratoire (2) et le volume d'air extérieur, durant la première phase respiratoire de sens direct, chaque alternance étant déclenchée par la détection que l'air est totalement renouvelé dans la première ou seconde chambre (T1, T2).

4. Procédé selon l'une des revendications 1 à 3, comprenant des étapes d'extinction des première et seconde sources (LL) de rayonnement ultraviolet pendant chaque phase respiratoire de sens inverse**.**

5. Procédé selon l'une des revendications 1 à 4, dans lequel :
les première et seconde phases respiratoires de sens direct sont des phases d'inspiration (INS) de l'utilisateur, et les première et secondes phase respiratoires de sens inverse sont des phases d'expiration (EXP) de l'utilisateur, ou bien
les première et seconde phases respiratoires de sens direct sont des phases d'expiration (EXP) de l'utilisateur, et les première et secondes phase respiratoires de sens inverse sont des phases d'inspiration (INS) de l'utilisateur.

6. Procédé selon l'une des revendications 1 à 5, comprenant des étapes d'activation d'un ventilateur (VT) pour faire circuler l'air durant chaque phase respiratoire de sens direct (INS).

7. Dispositif de désinfection d'air respiré par un utilisateur, le dispositif comprenant :
une première chambre (T1) et une seconde chambre (T2), en communication (I1, I2) avec un volume d'air extérieur et couplées par un dispositif de vanne (EV) commandé, à une première conduite (6) reliée au volume intérieur d'un masque respiratoire (2),
une source (LL) de rayonnement ultraviolet, installée à l'intérieur chacune des première et seconde chambres (T1, T2), et
un dispositif de mesure (PS, PRC) pour déterminer un sens de circulation de l'air dans la première conduite (6), le dispositif de désinfection étant configuré pour mettre en œuvre le procédé selon l'une des revendications 1 à 6, la détection des instants de début (t1-t8) de phases respiratoires d'inspiration (INS) et d'expiration (EXP) d'air étant effectuées à l'aide du dispositif de mesure (PS, PRC),
et les mises en circulation de l'air dans la première chambre (T1) et dans la seconde chambre (T2) étant effectuées à l'aide du dispositif de vanne (EV).

8. Dispositif selon la revendication 7, dans lequel chacune des première et seconde chambres (T1, T2) comprend un dispositif de ventilation (VT) pour faire circuler l'air au travers de la chambre (T1, T2).

9. Dispositif selon la revendication 8, dans lequel le dispositif de ventilation comprend une pompe ou un ventilateur (VT) et/ou met en œuvre l'effet Coanda.

10. Dispositif selon la revendication 7 ou 9, dans lequel la source (LL) de rayonnement UV dans chacune des première et seconde chambres (T1, T2) comprend une lampe à tube à quartz UV ou des LED UV.

11. Dispositif selon l'une des revendications 7 à 10, dans lequel le dispositif est installé dans un logement portatif, incluant une source d'alimentation électrique autonome (BT).

12. Dispositif selon l'une des revendications 7 à 11, dans lequel chacune des première et seconde chambre (T1, T2) est associée à un compresseur pour augmenter la pression dans la chambre (T1, T2).

13. Dispositif selon l'une des revendications 7 à 12, dans lequel la source (LL1) de rayonnement UV est installée dans chacune des première et seconde chambres (T1, T2) et configurée pour soumettre un volume d'exposition dans la chambre (T1, T2) à une intensité lumineuse supérieure ou égale à 10 mW/cm².

## Patentansprüche

1. Verfahren zum Desinfizieren der Luft, die durch einen Benutzer geatmet wird, das Verfahren umfassend die Schritte, bestehend aus:
Aussetzen des Innenraums einer ersten Kammer (T1), die in Verbindung (I1) mit einem Außenluftvolumen steht, einer ersten Quelle (LL) einer ultravioletten Strahlung, die eine Wellenlänge besitzt, die geeignet ist, um Keime zu zerstören,
Aussetzen des Innenraums einer zweiten Kammer (T2), die in Verbindung (11) mit dem Außenluftvolumen steht, einer zweiten Quelle (LL) der ultravioletten Strahlung,
Erfassen von Zeitpunkten (t1-t8) eines Starts von Atemphasen einer Einatmung (INS) und einer Ausatmung (EXP) von Luft, die durch den Benutzer erfolgen, wobei jede Atemphase mit einer Vorwärts- oder Gegenrichtung einer Zirkulation von Luft in einer Leitung (4, 6) während der Atemphase verknüpft ist,
während einer ersten Atemphase in Vorwärtsrichtung, die auf eine erste Atemphase in Gegenrichtung folgt, Zirkulierenlassen der Luft der ersten Kammer (T1) zwischen einem Innenvolumen einer Atemmaske (2), die den Mund und die Nase des Benutzers (1) dicht abdeckt, und dem Außenluftvolumen, wobei auf die erste Atemphase in Vorwärtsrichtung eine zweite Atemphase in Gegenrichtung folgt, und
**gekennzeichnet durch,**
während einer zweiten Atemphase in Vorwärtsrichtung, die auf die zweite Atemphase in Gegenrichtung folgt, Zirkulierenlassen der Luft der zweiten Kammer (T2) zwischen dem Innenvolumen der Atemmaske (2) und dem Außenluftvolumen.

2. Verfahren nach Anspruch 1, umfassend einen Schritt des Erfassens, dass die Luft der ersten Kammer (T1) vor dem Ende (t12, t25) der ersten Atemphase in Vorwärtsrichtung vollständig erneuert ist, gefolgt von einem Schritt des Zirkulierenlassens der Luft der zweiten Kammer (T2) abwechselnd mit dem Zirkulierenlassen der Luft der ersten Kammer (T1) zwischen dem Innenvolumen der Atemmaske (2) und dem Außenluftvolumen.

3. Verfahren nach Anspruch 1 oder 2, umfassend mehrere abwechselnde Schritte des Zirkulierenlassens der Luft der ersten Kammer (T1) und des Zirkulierenlassens der Luft der zweiten Kammer (T2) zwischen dem Innenvolumen der Atemmaske (2) und dem Außenluftvolumen, während der ersten Atemphase in Vorwärtsrichtung, wobei jeder Wechsel durch die Erfassung ausgelöst wird, dass die Luft in der ersten oder der zweiten Kammer (T1, T2) vollständig erneuert ist.

4. Verfahren nach einem der Ansprüche 1 bis 3, umfassend die Schritte eines Abschaltens der ersten und der zweiten Quelle (LL) von ultravioletter Strahlung während jeder Atemphase in Gegenrichtung.

5. Verfahren nach einem der Ansprüche 1 bis 4, wobei:
die erste und die zweite Atemphase in Vorwärtsrichtung Einatmungsphasen (INS) des Benutzers sind, und die erste und die zweite Atemphase in Gegenrichtung Ausatmungsphasen (EXP) des Benutzers sind, oder aber
die erste und die zweite Atemphase in Vorwärtsrichtung Ausatmungsphasen (EXP) des Benutzers sind, und die erste und die zweite Atemphase in Gegenrichtung Einatmungsphasen (INS) des Benutzers sind.

6. Verfahren nach einem der Ansprüche 1 bis 5, umfassend die Schritte eines Aktivierens eines Ventilators (VT), um die Luft während jeder Vorwärtsrichtungsatmungsphase (INS) in Zirkulation zu bringen.

7. Vorrichtung zum Desinfizieren von Luft, die durch einen Benutzer geatmet wird, die Vorrichtung umfassend:
eine erste Kammer (T1) und eine zweite Kammer (T2), die in Verbindung (I1, I2) mit einem Außenluftvolumen stehen und über eine gesteuerte Ventilvorrichtung (EV) mit einer ersten Leitung (6) gekoppelt sind, die mit dem Innenvolumen einer Atemmaske (2) gekoppelt ist,
eine Quelle (LL) von ultravioletter Strahlung, die jeweils im Inneren der ersten und der zweiten Kammer (T1, T2) installiert ist, und
eine Messvorrichtung (PS, PRC) zum Bestimmen einer Luftzirkulationsrichtung in der ersten Leitung (6), wobei die Desinfektionsvorrichtung zum Implementieren des Verfahrens nach einem der Ansprüche 1 bis 6 konfiguriert ist, wobei die Erfassung der Startzeitpunkte (t1-t8) der Einatmungs- (INS) und Ausatmungsatemphasen (EXP) von Luft, die mittels der Messvorrichtung (PS, PRC) durchgeführt wird, und das Zirkulierenlassen der Luft in der ersten Kammer (T1) und in der zweiten Kammer (T2) mittels der Ventilvorrichtung (EV) erfolgt.

8. Vorrichtung nach Anspruch 7, wobei jede der ersten und der zweiten Kammer (T1, T2) eine Ventilationsvorrichtung (VT) umfasst, um die Luft in Zirkulation durch die Kammer (T1, T2) zu bringen.

9. Vorrichtung nach Anspruch 8, wobei die Ventilationsvorrichtung eine Pumpe oder einen Ventilator (VT) umfasst und/oder den Coanda-Effekt implementiert.

10. Vorrichtung nach Anspruch 7 oder 9, wobei die Quelle (LL) von UV-Strahlung in jeder der ersten und der zweiten Kammer (T1, T2) eine UV-Quarzleuchtröhre oder UV-LEDs umfasst.

11. Vorrichtung nach einem der Ansprüche 7 bis 10, wobei die Vorrichtung in einem tragbaren Gehäuse installiert ist, einschließlich einer unabhängigen Stromversorgungsquelle (BT).

12. Vorrichtung nach einem der Ansprüche 7 bis 11, wobei jede der ersten und der zweiten Kammer (T1, T2) mit einem Kompressor zum Erhöhen des Drucks in der Kammer (T1, T2) verknüpft ist.

13. Vorrichtung nach einem der Ansprüche 7 bis 12, wobei die Quelle (LL1) von UV-Strahlung jeweils in der ersten und der zweiten Kammer (T1, T2) installiert ist und zum Aussetzen eines Expositionsvolumens in der Kammer (T1, T2) einer Lichtintensität von größer als oder gleich 10 mW/cm² konfiguriert ist.

## Claims

1. A method for disinfecting the air breathed in by a user, the method comprising steps of:
subjecting the inside of a first chamber (T1) in communication (11) with an outside air space, to a first source (LL) of ultraviolet radiation having a wavelength able to destroy germs,
subjecting the inside of a second chamber (T2) in communication (11) with the outside air space, to a second source (LL) of ultraviolet radiation,
detecting times (t1-t8) when the air inhalation (INS) and expiration (EXP) breathing phases carried out by the user start, each breathing phase being associated with a forward or backward direction of circulation in which air is circulated in a pipe (4, 6) during the breathing phase,
during a first forward direction breathing phase following a first backward direction breathing phase, circulating the air from the first chamber (T1)
between an inner volume of a breathing mask (2)
covering the mouth and the nose of the user (1) and the outside air space, the first forward direction breathing phase being followed by a second backward direction breathing phase, and wherein,
during a second forward direction breathing phase following the second backward direction breathing phase, circulating the air from the second chamber T2) between the internal space of the breathing mask (2) and the outside air space.

2. The method according to claim 1, comprising a step of detecting that the air of the first chamber (T1) is fully changed before the end (t12, t25) of the first forward direction breathing phase, followed by a step of circulating the air of the second chamber (T2) alternately with the circulating of the air from the first chamber (T1), between the internal space of the respiratory mask (2) and the outside air space.

3. The method according to claim 1 or 2, comprising several alternating steps to circulate the air from the first chamber (T1) and to circulate the air from the second chamber (T2), between the internal space of the respiratory mask (2) and the outside air space, during the first forward direction breathing phase, each alternation being triggered by the detection that the air is fully changed in the first or second chamber (T1, T2).

4. The method according to one of claims 1 to 3, comprising steps of turning off the first and second ultraviolet radiation sources (LL) during each backward direction breathing phase.

5. The method according to one of claims 1 to 4, wherein:
the first and second forward direction breathing phases are user inhalation phases (INS), and the first and second backward direction breathing phase are user expiration phases (EXP), or
the first and second forward direction breathing phases are user exhalation phases (EXP), and the first and second backward direction breathing phase are user inhalation phases (INS).

6. The method according to one of claims 1 to 5, comprising steps of activating a fan (VT) to circulate the air during each forward direction breathing phase (INS).

7. A device for disinfecting air breathed in by a user, the device comprising:
a first chamber (T1) and a second chamber (T2), in communication (11, I2) with an outside air volume and coupled by a controlled valve device (EV), to a first pipe (6) connected to the internal space of a breathing mask (2),
an ultraviolet radiation source (LL) installed inside each of the first and second chambers (T1, T2), and
a measurement device (PS, PRC) for determining a direction in which air circulates in the first pipe (6), the disinfecting device being configured to implement the method according to one of claims 1 to 6, the detection of the times (t1-t8) when the air inhalation (INS) and exhalation (EXP) phases start being carried out using the measuring device (PS, PRC), and the circulating of the air in the first chamber (T1) and in the second chamber (T2) being carried out using the valve device (EV).

8. The device according to claim 7, wherein each of the first and second chambers (T1, T2) comprises a ventilation device (VT) for circulating the air through the chamber (T1, T2).

9. The device according to claim 8, wherein the ventilation device comprises a pump or a fan (VT) and/or implements the Coanda effect.

10. The device according to claim 7 or 9, wherein the source (LL) of UV radiation in each of the first and second chambers (T1, T2) comprises a UV quartz tube or UV LED lamp.

11. The device according to one of claims 7 to 10, wherein the device is installed in a portable housing, including an autonomous electric power source (BT).

12. The device according to one of claims 7 to 11, wherein each of the first and second chamber (T1, T2) is associated with a compressor to increase the pressure in the chamber (T1, T2).

13. The device according to one of claims 7 to 12, wherein the UV radiation source (LL1) is installed in each of the first and second chambers (T1, T2) and configured to subject an exposure volume in the chamber (T1, T2) to a light intensity greater than or equal to 10 mW/cm².
